Europäisches Patentamt

European Patent Office

Office européen des brevets

(19)

(11) Numéro de publication : **0 454 553 A1**

(12)

# DEMANDE DE BREVET EUROPEEN

(21) Numéro de dépôt : **91401053.3**

(51) Int. Cl.⁵ : **A61D 1/06, A61F 6/20**

(22) Date de dépôt : **19.04.91**

(30) Priorité : **25.04.90 FR 9005499**

(43) Date de publication de la demande :
**30.10.91 Bulletin 91/44**

(84) Etats contractants désignés :
**AT BE CH DE DK ES FR GB GR IT LI LU NL SE**

(71) Demandeur : **Berten, David**
**La Raye**
**F-44140 Montbert (FR)**
Demandeur : **Neau, François-Xavier**
**"La Ville en Bois", Getigne**
**F-44190 Clisson (FR)**

(72) Inventeur : **Berten, David**
**La Raye**
**F-44140 Montbert (FR)**
Inventeur : **Neau, François-Xavier**
**"La Ville en Bois", Getigne**
**F-44190 Clisson (FR)**
Inventeur : **Herbert, René**
**Soullans**
**F-85300 Challans (FR)**

(74) Mandataire : **Le Brusque, Maurice et al**
**Cabinet Harlé et Phélip 21, rue de la**
**Rochefoucauld**
**F-75009 Paris (FR)**

(54) **Matériel de stérilisation d'animal femelle par ischemie et clip de stérilisation.**

(57)    Le matériel de stérilisation d'animal femelle par ischémie et dégénérescence du tissu ovarien, comprend des moyens pour provoquer l'ischémie, en forme de clip (2', 2") et un instrument de pose dudit clip en forme de manche (1). Ce manche est muni, à l'une de ses extrémités, d'une tête opératoire destinée à être prise par une main de l'opérateur, in situ, et, à son autre extrémité, de moyens de manoeuvre (5, 6) à distance du clip (2', 2"), actionnés par l'autre main de l'opérateur, depuis l'extérieur.
    Ce matériel peut avantageusement être utilisé pour la stérilisation de femelles de bovins, en fin de lactation, pour améliorer les quantités et qualités de viande.

EP 0 454 553 A1

_fig.1_

_fig.2_

La présente invention concerne le domaine vétérinaire ; elle a plus particulièrement pour objet un matériel destiné à la stérilisation par ischémie d'animaux femelles.

En dehors de l'aspect curatif, la stérilisation d'animaux femelles peut présenter de nombreux avantages. En particulier, pour les vaches de réforme qui terminent une lactation, la modification des cycles hormonaux, liée à cette stérilisation, permet d'améliorer les quantités et qualités de la viande, apportant par là même, une plus value économique non négligeable. Les gains en poids peuvent ainsi être de l'ordre de 7 à 8 % ; la stérilisation permet également d'orienter les métabolismes de l'animal vers une viande plus rouge, plus tendre, peu grasse et plus goûteuse.

Différentes techniques ont été proposées pour réaliser la stérilisation de l'animal. En règle générale, le technicien opère en aveugle, soit par les flancs après incision cutanée, soit, de façon préférée, car plus rapide et plus aisée, par la voie vaginale, après perforation de la paroi vaginale.

L'opérateur introduit l'une de ses mains jusqu'aux ovaires et le matériel opératoire est introduit simultanément ou ultérieurement ; ce matériel est adapté à un travail quasiment en aveugle, d'une seule main. Dans tous les cas, et pour des raisons d'aseptie, on s'efforce de réduire au minimum les pénétrations jusqu'à l'organe à opérer, sous peine de multiplier les risques d'infection.

Les techniques par écraseurs, ou les techniques d'ovariectomie qui consistent en une exérèse de l'ovaire, sont délicates à réaliser et elles sont souvent longues et stressantes pour l'animal. Ces techniques présentent en outre les risques classiques d'accident opératoire, tels que des hémorragies, liées à toute intervention chirurgicale. Le matériel utilisé pour ce type d'opération, tel par exemple celui décrit dans le document AU-B-28200/77, est souvent lourd et peu maniable ; il doit être stérilisé entre chaque opération afin d'éviter les risques d'infection.

Il est également connu, dans le domaine de la médecine vétérinaire, d'utiliser des techniques d'ischémie, c'est-à-dire d'arrêt de la circulation sanguine pour provoquer la dégénérescence du tissu ovarien et en conséquence la stérilisation de l'animal.

Ces techniques sont mises en oeuvre au moyen d'anneaux de DEGIVE, lesquels anneaux consistent en une bague de caoutchouc enfilée sur une perle creusée d'un trou conique. Après perforation de la paroi vaginale, l'anneau est mis en place autour du pédicule ovarien et il est serré sur ce dernier au moyen de la perle pour réaliser l'ischémie par ligature.

Ce travail est réalisé d'une seule main, en aveugle. L'opérateur saisit l'anneau entre trois doigts, à l'extérieur, il le maintient en tension et l'introduit par la vulve de l'animal. Une cordelette, qui passe dans la boucle arrière de l'anneau, permet, par une traction de l'extérieur avec la main libre, de faciliter le serrage au moyen de la perle. Cette technique est très délicate à réaliser ; elle présente également des risques d'infection, et notamment de péritonite, liés en particulier à la manipulation de la cordelette de manoeuvre, non stérile, qui transite par la vulve souvent souillée d'excréments, et par la cavité abdominale.

Dans un autre domaine, on connaît du document EP-A-0106748 un appareil pour la pose de clip chirurgical destiné à l'hémostase d'un vaisseau sanguin. Cet appareil permet au chirurgien de poser le clip, à vue ; l'opération est réalisée d'une seule main, après avoir dégagé l'ensemble des tissus environnants.

Etant données les conditions opératoires bien particulières du domaine technique correspondant à l'invention, les dimensions et caractéristiques du matériel décrit dans le document EP-A-0106748, ne sont en aucun cas adaptées ni prévues pour permettre ou envisager une opération de stérilisation tel qu'on se le propose dans la présente demande, c'est-à-dire une opération dans laquelle on doit progresser en aveugle à travers les tissus de l'animal et dans laquelle il est nécessaire d'emprisonner et de pincer un amas de tissus vasculaires.

L'invention a pour but de pallier à ces différents inconvénients et elle propose un matériel de stérilisation par ischémie offrant de bonnes garanties d'efficacité et réduisant sensiblement les problèmes d'aseptie et les risques hémorragiques liés aux techniques antérieures. Les caractéristiques structurelles du matériel le rendent simple à construire, léger, maniable et peu encombrant. Ce matériel est utilisable avec le minimum de personnel en aide extérieure, il peut de façon très avantageuse, se présenter stérile et être à usage unique pour supprimer la désinfection entre chaque animal ; cet usage unique permet également d'éliminer les problèmes de transmission de certaines maladies virales par voie sanguine (leucose, IPR, PVD ...).

Le matériel de stérilisation d'animal femelle selon l'invention comprend des moyens en forme de clip pour obturer par pincement l'ensemble des voies sanguines qui irriguent les ovaires. Il comprend également un instrument de pose du clip, muni, à l'une de ses extrémités d'une tête opératoire destinée à être prise par une des mains de l'opérateur, in situ, et, à son autre extrémité, de moyens de manoeuvre à distance du clip, actionnés par l'autre main de l'opérateur, depuis l'extérieur. Toujours selon l'invention, le clip est constitué de deux bras pinceurs reliés entre eux, à une de leurs extrémités, par une articulation, et munis à leur autre extrémité, de moyens de solidarisation destinés au maintien dudit clip en position fermée de serrage. Le clip est associé à un instrument de pose comprenant un support allongé en forme de manche, muni à l'une de ses extrémités, de moyens de maintien du clip en position ouverte, prêt à être positionné autour du pédicule ovarien. Ce manche a une longueur $\underline{L}$ adaptée pour permettre à l'opérateur de manoeuvrer depuis l'extérieur, pour amener le clip jusqu'aux ovaires et en particulier jusqu'aux pédicules ovariens à traiter.

Selon une autre caractéristique, la tête du manche allongé définit un renflement d'extrémité en forme de Vé, lequel Vé est constitué par deux extensions latérales divergentes qui maintiennent chacune un bras du clip. Cette caractéristique est très avantageuse pour permettre la progression à travers les tissus et pour proposer une sorte de butée d'extrémité pour la main de l'opérateur qui guide la tête du matériel. En effet, ce dispositif est destiné en majeure partie à être utilisé en aveugle ; la tête large limite la grosseur du manche et permet de dégager les tissus autour du site opératoire.

Selon une autre caractéristique, le manche allongé est creux et renferme un poussoir, commandé de l'extérieur, apte à au moins amorcer le positionnement du clip sur l'organe à traiter.

De préférence, les extensions latérales qui définissent la tête du manche comportent des paires de lèvres qui prolongent la cavité interne dudit manche et qui servent d'organe de guidage et d'encastrement des bras du clip. L'extrémité de ces extensions est munie de guides en forme de cames qui servent à la commande de la fermeture des bras du clip asservi au poussoir.

De façon avantageuse, le poussoir est commandé de l'extérieur, au moyen d'une gâchette en saillie disposée au niveau de l'extrémité du manche opposée à la tête support de clip. Cette gâchette coopère avec un système de prise, en forme de butée fixe sur le manche, pour la manoeuvre d'une seule main par l'opérateur.

Pour limiter les introductions de matériel opératoire au niveau de l'ouverture péritonéale, le manche support peut avantageusement être associé à deux clips positionnés l'un derrière l'autre et asservis tous deux au poussoir. Cette caractéristique permet le traitement des deux glandes, successivement, sans avoir à ressortir le matériel et le bras chirurgical. Dans ce cas, le poussoir comporte de préférence une gâchette de manoeuvre à deux positions, associée à des moyens anti-retour du poussoir par rapport au manche.

Selon d'autres caractéristiques préférées, le manche et le ou les clips associés sont réalisés par moulage de matériau plastique biocompatible du genre PVC ; la longueur du manche est de l'ordre de 30 à 80 cm. Le support de clip peut également être associé à un perforateur destiné par exemple à la ponction de la paroi vaginale. Ce perforateur est de préférence amovible et il vient coiffer l'extrémité du manche.

Le matériel peut encore avantageusement être muni d'une lèvre complémentaire, moulée à l'intérieur du support et servant de cran de sécurité pour le mouvement du poussoir ; le support allongé, rigide, permet de positionner les moyens opératoires en forme de clips, à volonté, par guidage et manoeuvre, avec la main libre, depuis l'extérieur ; la progression et la manoeuvre de la main opératrice, in situ, s'en trouvent facilitées. Cette nouvelle technique est plus facile à mettre en oeuvre et plus simple que les techniques antérieures, elle est aussi plus fiable et limite les risques d'infection.

L'invention concerne également un clip destiné notamment mais non exclusivement à être adapté sur l'instrument de pose en forme de manche et qui permet de réaliser la stérilisation d'animaux femelles par pincement du pédicule ovarien. Le clip comporte des bras pinceurs reliés entre eux par une articulation et munis, à leur autre extrémité, de moyens de solidarisation destinés à leur maintien en position fermée de serrage. Le clip comporte également, à chacune de ses extrémités, des moyens en forme de parois destinés à former, avec les bras, un espace clos dans lequel sont emprisonnés les tissus avant pincement.

Ces caractéristiques permettent au clip de définir une sorte d'anneau s'apparentant à l'anneau de DEGIVE, de l'état de la technique. La section close évolutive obtenue est, avant fermeture de l'espace disponible, supérieure à la section des tissus emprisonnés.

Toujours selon l'invention, le clip comporte une articulation flexible en forme d'anneau qui relie l'une des extrémités des deux bras ; cette articulation est associée à une languette de blocage des tissus et, éventuellement, de rappel élastique. L'autre extrémité de ces bras est munie, pour l'une, d'appendices mâles, et pour l'autre, d'échancrures femelles, destinés à l'obtention d'une pluralité de crans de solidarisation. Ces moyens de solidarisation sont destinés au maintien du clip en position fermée de serrage. Ce serrage est réglable et peut être adapté aux différentes tailles d'ovaires à traiter. De préférence, la zone médiane de ces moyens de solidarisation est pourvue d'éléments mâles et femelles complémentaires qui évitent le déport latéral des bras l'un par rapport à l'autre.

Selon une autre caractéristique de l'invention, les faces internes en vis à vis des bras du clip sont crantées transversalement ; l'une de ces faces comporte une barrette longitudinale qui fait saillie et qui coopère avec une échancrure longitudinale prévue dans l'autre face pour former une chicane.

De préférence, la face interne de l'un des bras pinceurs comporte des moyens d'emprisonnement et de blocage de la progression des tissus lors du pincement. Ces moyens sont en forme de butée faisant saillie vers le bras homologue ; cette butée est disposée légèrement en amont des moyens de solidarisation d'extrémité et elle coopère avec une réservation de forme complémentaire réalisée dans le bras homologue pour permettre la fermeture.

De façon avantageuse, la butée a une forme triangulaire ; sa face arrière est destinée au blocage des tissus et sa face avant comporte deux lèvres latérales qui définissent un canal dans lequel vient s'encastrer une lèvre médiane prévue dans sa réservation homologue, lors du serrage des bras.

Toujours selon l'invention, les extrémités libres des bras du clip comportent des tétons en saillie qui sont destinés à coopérer avec des rainures prévues dans le manche support et dans ses deux extensions d'extrémité, pour le guidage dudit clip asservi au poussoir de positionnement. La partie arrière du clip est également munie d'un appendice, disposé au niveau de l'articulation ; cet appendice, en saillie, permet, d'une part, le guidage de l'arrière du clip dans une rainure prévue à l'intérieur du manche, et, d'autre part, le positionnement du clip entre deux butées latérales prévues à l'extrémité du poussoir.

Mais l'invention sera encore illustrée, sans être aucunement limitée par la description suivante d'un mode de réalisation particulier, donné uniquement à titre d'exemple et représenté sur les dessins annexés dans lesquels :

      – la figure 1 représente la partie avant du manche support de clip, vue de côté, en coupe longitudinale, avec des sections de détails ;

      – la figure 2 est une vue de côté, en coupe longitudinale, de la partie arrière du manche, le poussoir interne étant représenté dans une position arrière extrême, avec une coupe simplifiée au niveau du poussoir ;

      – la figure 3 représente la partie arrière du manche support avec le poussoir interne dans une position moyenne, avec une coupe de la partie arrière du manche et du poussoir ;

      – la figure 4 est une vue de côté du clip selon l'invention ;

      – la figure 5 est une vue du clip, en coupe selon 5-5 ;

      – la figure 6 est une vue en bout du bras supérieur tel que représenté figure 4 ;

      – la figure 7 est une vue du clip en coupe selon 7-7 ;

      – la figure 8 est une vue du clip en coupe selon 8-8 ;

      – la figure 9 est une vue schématique du clip selon l'invention, en position intermédiaire de serrage, montrant le pincement du pédicule ovarien et le blocage des tissus par la butée intégrée ;

      – les figures 10 et 11 représentent le perforateur amovible destiné à être adapté sur la tête d'extrémité avant du manche.

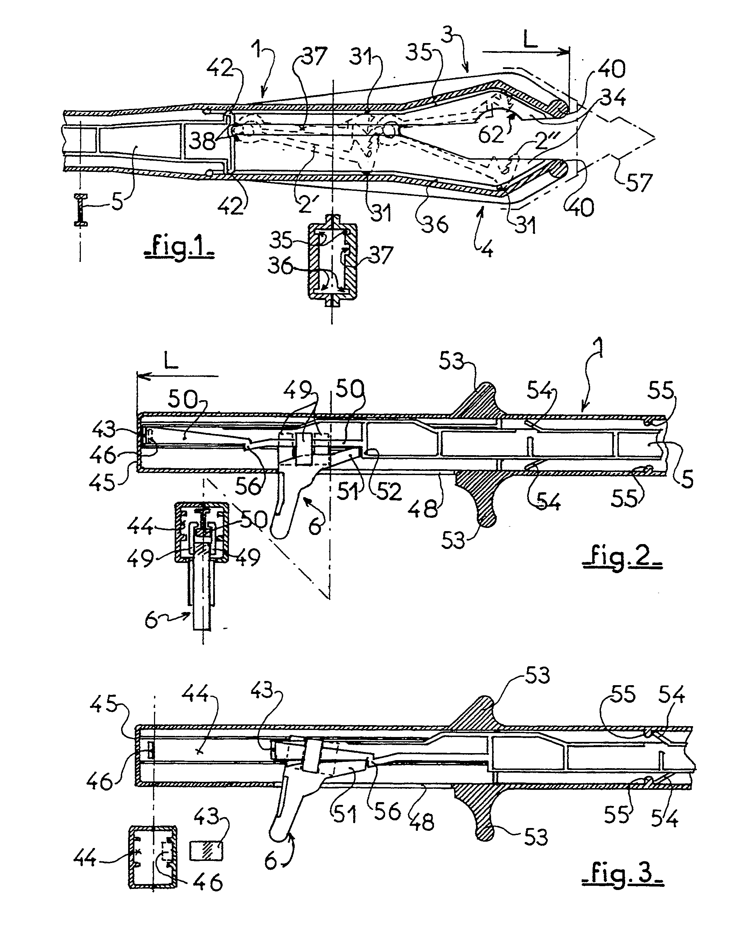

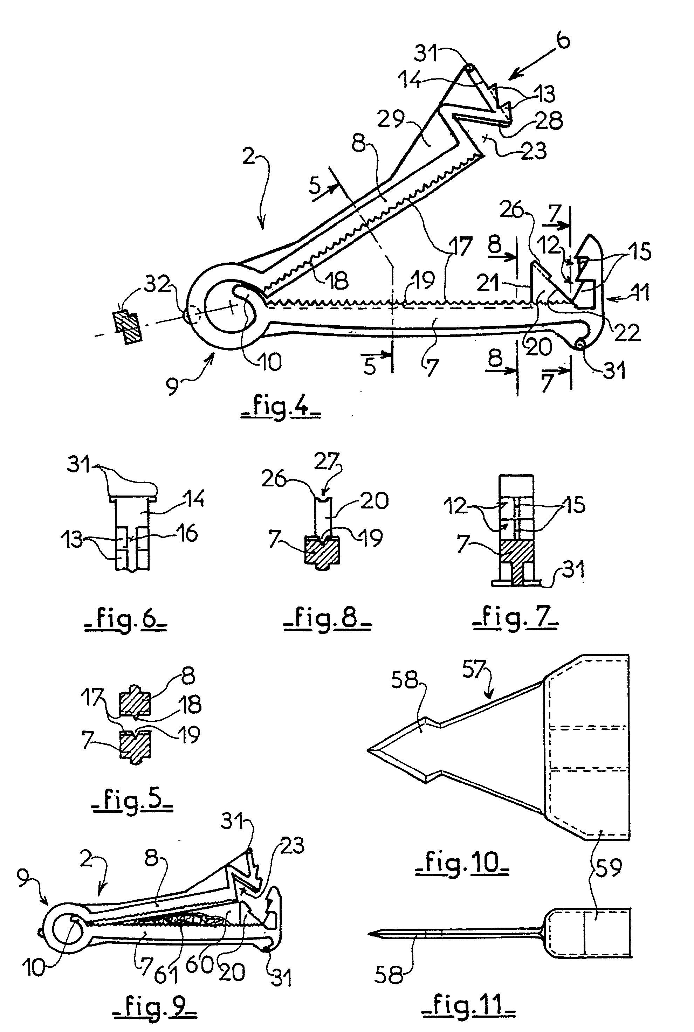

Tel qu'on l'a représenté figures 1 et 2, le dispositif selon l'invention consiste en un support allongé 1 en forme de manche rigide qui est muni, à l'une de ses extrémités, de moyens de maintien et de positionnement de deux clips 2', 2" représentés en pointillés. La partie avant du manche 1 est représentée figure 1 ; la figure 2 montre sa partie arrière. La longueur $\underline{L}$ du manche 1 doit permettre à un opérateur d'amener, de l'extérieur, le ou les clips 2', 2" jusqu'aux ovaires à traiter, par la voie vaginale ou par les flancs. Pour des femelles de bovins, le manche 1 peut avoir une longueur de l'ordre de 30 à 80 cm, de préférence 50 à 70 cm.

Le matériel représenté est constitué d'un manche creux dont la partie arrière a une forme sensiblement rectangulaire et dont la partie avant s'élargit progressivement pour déterminer un renflement d'extrémité. Comme on peut le voir sur la figure 1, cette partie avant est en forme de Vé constitué de deux extensions latérales divergentes 3 et 4 qui définissent un espace conformé selon la structure du clip 2', 2" à réceptionner.

Le support 1 est de préférence réalisé par moulage de matériau plastique biocompatible du type PVC ; il est de préférence obtenu par assemblage de deux demi-coquilles homologues.

La cavité interne du manche 1 renferme un poussoir longitudinal 5 guidé par les parois du manche et/ou au moyen de rainures internes. Le poussoir coulissant 5 est associé à des moyens de commande en forme de gâchette 6 qui assure son mouvement longitudinal à l'intérieur du manche et qui sera détaillée plus loin.

Le poussoir 5 agit sur la base du ou des clips 2 et permet leur sortie du manche support 1 pour permettre leur positionnement sur l'organe à traiter. Le mouvement du ou des clips 2', 2" est également asservi à des moyens de guidage prévus au niveau de la partie avant du manche 1 de façon à assurer un positionnement correct et aisé sur l'organe à traiter. Ces moyens de guidage seront détaillés plus loin.

Un exemple de réalisation d'un clip 2 prévu pour être monté sur le manche 1 est représenté figures 4 à 9. Ce clip 2 définit une mâchoire constituée de deux bras pinceurs, ou mors 7 et 8, reliés entre eux, à une de leurs extrémités, au moyen d'une articulation 9 ; sa structure est particulièrement adaptée à sa fonction de pose sur le pédicule ovarien par l'intermédiaire d'un manche support. Cependant d'autres applications de ce clip particulier pourront être envisagées en liaison avec les avantages que sa structure particulière peut apporter. Le clip 2 est avantageusement réalisé en matériau plastique biocompatible tel que du PVC de façon monobloc. L'articulation 9 est obtenue par formation d'une boucle ouverte ; la structure du clip et la nature du matériau utilisé rendent cette articulation flexible. En position repos, les bras 7 et 8 divergent à partir de l'articulation 9 et le clip 2 est ouvert. Pour le fermer et positionner les deux bras 7 et 8 parallèlement l'un à l'autre, il est nécessaire d'appliquer une pression latérale sur chacun desdits bras. Une languette monobloc 10 est adaptée sur l'articulation 9 pour obturer l'espace entre les bras 7 et 8 et bloquer la progression des tissus de ce côté ;

la languette 10 sert également de moyen de rappel élastique dans le cas où la flexibilité de l'articulation 9 n'est pas suffisante.

L'extrémité libre des bras 7 et 8 comporte des moyens de solidarisation destinés au maintien du clip en position fermée, c'est-à-dire en position de serrage. Le bras 7 est prolongé par une patte 11 qui s'étend sensiblement en équerre, vers le bras homologue 8. Cette patte 11 est munie d'un ensemble d'échancrures femelles internes 12 qui, dans l'exemple représenté, sont au nombre de deux. Les échancrures femelles 12 sont destinées à coopérer avec les appendices mâles 13 conformés à l'extrémité du bras 8. Ces appendices 13, également au nombre de deux, sont disposés sur une patte 14 qui s'étend en équerre, vers l'extérieur, à l'opposé du bras homologue 7. Les appendices 13 sont orientés dans la direction de l'axe du bras 8 et sont destinés à venir s'engager dans les orifices femelles 12 de la patte 11 pour servir de moyens de fermeture du clip 2. Cette fermeture est obtenue par rapprochement des deux bras 7 et 8 l'un vers l'autre ; la pluralité d'échancrures femelles 12 et d'appendices mâles 13 permet d'obtenir une solidarisation réglable, en forme de crans, pour l'adaptation du clip selon l'épaisseur du pédicule ovarien à traiter. Le nombre et la forme des crans peut varier suivant les réglages désirés en particulier.

La patte 11 qui s'étend en équerre à l'extrémité du bras 7 comporte également, figures 4 et 7, deux lèvres médianes 15 qui s'étendent transversalement aux échancrures femelles 12. Ces lèvres médianes 15 sont destinées à coopérer, lors de la fermeture du clip, avec une réservation médiane 16 prévue sur la face correspondante de la patte homologue 14. La pénétration des lèvres 15 dans la réservation 16 conforte le positionnement des deux bras 7 et 8 l'un par rapport à l'autre et évite notamment leur déport latéral lorsque des efforts importants de pincement leur sont soumis.

La fermeture du clip 2 réalise le rapprochement des deux bras 7 et 8 dont les faces internes vont venir se positionner l'une contre l'autre ou à proximité l'une de l'autre. Afin d'améliorer le pincement, les faces internes de chaque bras 7 et 8 en vis à vis sont crantées et comportent ainsi une suite d'échancrures transversales 17 qui définissent une série de parties mâles et femelles alternées. Ces parties mâles et femelles sont décalées d'un bras sur l'autre de façon à ce que les parties mâles d'un bras coopèrent avec les parties femelles de l'autre bras. En position de serrage, on réalise ainsi un plan de joint en forme de ligne brisée.

La face interne du bras 8 comporte en outre une barrette longitudinale 18, faisant saillie, disposée sensiblement sur la médiane de ladite face. Cette barrette 18 est destinée à venir s'encastrer dans une échancrure longitudinale 19 prévue dans la face interne correspondante du bras 7 pour former une chicane.

Cette structure particulière permet de réaliser une double rupture de la circulation sanguine au niveau du pédicule ovarien. Cette caractéristique permet d'améliorer la qualité de pincement des bras et complète les moyens 15 et 16 précités qui évitent le déport latéral desdits bras l'un par rapport à l'autre. Comme on peut le voir sur la figure 4, la barrette longitudinale 18 a une hauteur qui s'accroît légèrement de l'avant vers l'arrière. La différence de hauteur d'un point d'extrémité à l'autre est de l'ordre de 2/10ème de mm. Cette caractéristique permet d'améliorer le pincement sur l'organe à traiter, au niveau de la zone arrière du clip, c'est-à-dire du côté de l'articulation, laquelle zone a tendance à se rouvrir légèrement lorsque le bras 8 se courbe sous l'effet de l'épaisseur du pédicule ovarien écrasé, au moment de la fermeture de la pince.

La face interne du bras 7 comporte également une butée 20 qui fait saillie vers le bras homologue 8. Cette butée 20 a une forme sensiblement triangulaire et elle est disposée légèrement en amont de la patte d'extrémité 11. Elle comporte une face arrière 21 qui s'étend sensiblement perpendiculairement au bras 7 et une face avant 22, inclinée, qui s'étend jusqu'à la base de la patte 11. Comme on peut le voir figure 8, la butée 20 ne s'étend pas sur toute la largeur du bras 7 ; sa largeur est de l'ordre du tiers de celle du bras 7 ; elle est disposée sur ce bras selon une position médiane. Le bras homologue 8 comporte une réservation 23 de forme complémentaire triangulaire destinée à l'encastrement de la butée 20 lors du serrage.

La butée 20 a une hauteur qui correspond sensiblement à la largeur du bras 7, c'est-à-dire de l'ordre de 6 mm ; elle permet d'éviter la progression des tissus vers l'extrémité du clip lors du serrage des bras. Cet effet est représenté schématiquement figure 9. La butée 20 a des dimensions telles que son extrémité supérieure arrive sensiblement au niveau de la face interne du bras 8, juste avant le premier crantage de solidarisation. Les faces internes des deux bras 7 et 8, associées à la face arrière 21 de la butée 20, définissent ainsi un espace fermé duquel les tissus du pédicule ovarien ne peuvent s'échapper. On évite ainsi les risques éventuels d'éviction d'une partie du tissu hors de la zone de serrage du clip, risques qui permettraient la poursuite de l'irrigation sanguine des ovaires.

La face avant 22 de la butée 20 comporte également deux lèvres latérales 26, visibles figures 4 et 8. Ces deux lèvres 26 définissent un canal 27 dans lequel va venir s'encastrer une lèvre médiane 28 prévue sur la face correspondante de la réservation 23. Cette caractéristique améliore encore le positionnement et le centrage des bras 7 et 8 l'un par rapport à l'autre.

La nature du matériau utilisé et la structure du clip sont adaptées pour assurer un positionnement aisé et efficace du clip sur le pédicule ovarien à traiter. Dans ce but, la patte 11 disposée à l'extrémité du bras 7 présente une légère flexibilité dans le plan médian du clip ; cette caractéristique permet de faciliter l'encastrement des organes mâles 13 en forme de crochets. La partie correspondante du bras 8 est rigidifiée au moyen de nervures de renforcement 29 qui permettent d'améliorer la tenue de la patte 14.

Les extrémités libres des bras 7 et 8 comportent également des tétons latéraux 31, disposés aux extrémités externes respectives des pattes 11 et 14. Ces tétons monobloc 31 font saillie latéralement par rapport au clip, vu de face (figures 6, 7 et 8) ; ils sont destinés à venir se positionner dans des rainures réalisées dans la face interne du manche support 1 pour guider correctement le clip lors de sa progression à l'extrémité dudit manche.

La base du clip comporte également un appendice d'extrémité 32 qui fait saillie, d'une part, latéralement par rapport au plan des bras 7 et 8 et, d'autre part, vers l'arrière par rapport à l'articulation 9. Cet appendice 32 est destiné à venir s'encastrer dans une rainure correspondante réalisée sur l'une des faces internes du manche 1, pour le guidage de la partie arrière du clip 2 lors de sa progression ; cet appendice sert également au positionnement de la base du clip 2 sur l'extrémité du poussoir 5.

Le clip 2 a des dimensions adaptées au pincement d'un amas tissulaire vascularisé. Pour des femelles de bovins, les bras 7 et 8 peuvent avoir une longueur de l'ordre de 5 à 8 cm, une largeur de 0,5 à 1 cm et une épaisseur de l'ordre de 0,5 cm.

La structure particulière du clip, comme représenté figure 9, avec deux bras 7 et 8 rigides, associés à des parois ou butées d'extrémité 10 et 20 permet d'obtenir un espace clos 60 juste avant le verrouillage des mâchoires d'extrémité ; cet espace a alors une surface sensiblement supérieure à la section de l'amas tissulaire 61. Le rapprochement des bras entraîne une diminution de l'espace disponible et le pincement progressif des tissus, sans que ces derniers puissent s'échapper de cet espace clos.

Tel qu'on peut le voir sur la figure 1, le support 1 renferme, à l'arrière, le poussoir 5 destiné à agir sur les deux clips 2', 2″ disposés à la suite l'un de l'autre, dans la partie avant. Cette extrémité avant est adaptée pour au moins amorcer le positionnement des clips sur le pédicule ovarien à traiter. Les extensions latérales 3 et 4 comportent chacune des paires de lèvres de guidage 34, dirigées vers l'intérieur. Ces paires de lèvres prolongent la cavité du manche et s'étendent jusqu'aux extrémités desdites extensions 3 et 4.

Le premier clip 2' est positionné à l'intérieur du manche 1, en butée sur le poussoir 5. Le deuxième clip 2″ est prêt à être utilisé ; il est logé dans le Vé porteur défini par les deux extensions latérales 3, 4 ; il est maintenu en place par les lèvres de guidage 34 et sa base est en appui sur l'extrémité avant du premier clip 2'.

Les clips 2' et 2″ sont asservis au mouvement du poussoir coulissant 5 et leur guidage dans le support 1 est réalisé, d'une part, au moyen de leurs tétons d'extrémité 31 qui s'encastrent dans des rainures 35 et 36 correspondantes et, d'autre part, au moyen de l'appendice arrière 32, qui, lui-même, s'encastre dans une rainure 37 interne appropriée. Cet appendice arrière 32 permet également le positionnement du clip 2″ sur le poussoir 5, entre les deux butées latérales 38 d'extrémité, ainsi que le positionnement du clip avant 2″ sur l'extrémité du bras 8 du clip arrière 2', par encastrement entre les échancrures mâles 13.

Les rainures de guidage 35, 36 et 37 s'étendent sur toute la longueur de la course des clips 2' et 2″, entre l'extrémité du poussoir 5, tel que positionné figure 1, et l'extrémité avant du manche 1. Ces rainures suivent des trajets déterminés en fonction du mouvement relatif des bras 7 et 8 que l'on désire obtenir. La rainure 37, qui guide la base des clips 2' et 2″, est disposée sensiblement dans l'axe du Vé d'extrémité ; elle est rectiligne et s'arrête au niveau de la base du Vé porteur. Les deux rainures 35 et 36 sont disposées de chaque côté de la rainure 37 et suivent un parcours sensiblement symétrique. Ces deux rainures sont sensiblement parallèles jusqu'à la base du Vé porteur ; à partir de ce niveau elles commencent à diverger pour atteindre un maximum sensiblement au niveau des 3/4 de la longueur des extensions latérales 3 et 4 ; elles convergent ensuite jusqu'aux extrémités correspondantes du manche 1. Ces extrémités définissent des guides 40 en forme de cames qui permettent le rapprochement des bras des clips, en fin de course. Les guides 40 sont adaptés aux formes et encombrements des clips ; ils peuvent, selon le cas, permettre la fermeture de ces clips simplement au premier cran par l'action seule du poussoir 5, ou réaliser un serrage complet d'emblée. La fermeture complète et définitive du clip peut être réalisée manuellement, par l'opérateur, avec sa main qui se situe à l'intérieur.

Le mode de réalisation représenté montre deux clips 2' et 2″ positionnés l'un derrière l'autre et asservis tous les deux au poussoir 5 ; on dispose ainsi, en une seule introduction du manche de tout le matériel nécessaire au traitement des deux ovaires.

Le poussoir 5 commande par coulissement le déplacement et la pose des clips d'extrémité. Ce poussoir est lui-même guidé dans le corps du manche, à l'avant, au moyen d'extensions latérales 42 qui viennent en butée avec un léger jeu sur la paroi du manche 1 et, à l'arrière, par l'intermédiaire d'un guide d'extrémité 43 qui coulisse dans une rainure longitudinale 44 aménagée dans les parois latérales dudit manche

Avant l'emploi, le poussoir 5 est positionné complètement à l'arrière du manche 1, en butée sur sa base 45. A ce niveau, le support 1 peut avantageusement comporter une lèvre ou languette monobloc 46 coopérant avec le guide 43 et faisant office de cran de sécurité pour éviter le déplacement involontaire du poussoir 5 et la sortie du clip d'extrémité. Cette lèvre 46 est réalisée de façon à pouvoir être cassée par le guide 43 lors d'une poussée volontaire sur le poussoir 5.

Le déplacement du poussoir 5 est commandé par l'intermédiaire d'une gâchette 6 active au niveau de son extrémité arrière et qui s'étend en saillie à l'extérieur du support, par un orifice longitudinal 48. La gâchette 6 est mobile par rapport au poussoir 5 ; elle comporte des ergots 49 disposés de part et d'autre de l'âme centrale dudit poussoir 5 et qui prennent appui sur une rampe 50 conformée au niveau de la partie arrière du poussoir 5 et qui permettent son guidage sur ledit poussoir.

En position d'attente de pose du premier clip 2″, la gâchette 6 est positionnée à l'extrémité arrière de l'orifice longitudinal 48 ; sa partie avant 51 est en butée sur un retour en équerre 52 conformé sur le poussoir 5 (figure 2). On remarque qu'un déplacement vers l'avant de la gâchette 6 par rapport au manche 1 réalise le coulissement vers l'avant du poussoir 5. On a prévu sur le manche 1 des butées fixes monobloc 53 qui permettent cette manoeuvre d'une seule main. La longueur de l'ouverture 48 par laquelle passe la gâchette 6 correspond à une course du poussoir 5 qui permet la pose d'un clip sur l'organe à traiter.

Le poussoir 5 comporte des moyens anti-retour en forme de lèvres 54 disposées de façon appropriée sur sa longueur. Ces lèvres 54 sont destinées à coopérer avec des butées 55 prévues dans la cavité du manche de façon à éviter un retour en arrière du poussoir 5 une fois le premier clip 2″ posé. Cette position anti-retour est représentée figure 3. La flexibilité des lèvres 54, orientées convenablement, permet leur passage sur les butées 55 mais une fois passées elles interdisent le retour du poussoir vers l'arrière. La gâchette 6 est arrivée en bout de course et peut alors être ramenée vers l'arrière par coulissement des ergots 49 sur la rampe 50. Comme on peut le voir figures 2 et 3, la rampe 50 s'élève vers l'arrière, et, lors du coulissement de la gâchette, entraîne le basculement de cette dernière qui peut ainsi franchir, légèrement à force, le second retour en équerre 56 prévu sur le poussoir. Ce retour 56 sert de deuxième butée pour la partie avant 51 de la gâchette 6 et cette dernière va pouvoir recommencer son cycle pour le positionnement du deuxième clip 2′.

Afin de mettre à la disposition du technicien l'ensemble instrumental lié à l'opération, le support 1 peut être avantageusement associé à un perforateur 57 représenté figure 10 et en pointillés figure 1. Ce perforateur 57 consiste en une simple pièce plastique moulée, constituée d'une lame 58 munie de bordures coupantes, montée sur une coiffe 59. Il vient s'emmancher, tel un capuchon, sur les extrémités latérales 3 et 4 du manche 1.

L'opération de stérilisation est réalisée par les flancs de l'animal, au moyen d'un bistouri, ou par la voie vaginale en réalisant la ponction de la paroi vaginale avec le perforateur 57. La pénétration de la main de l'opérateur est réalisée simultanément ou un peu avant l'introduction du clip 2 associé à son manchesupport 1, lequel est guidé de l'extérieur par la main libre du technicien. Le matériel est alors dans la position représentée figures 1 et 2 ; le poussoir 5 et la gâchette 6 de commande sont dans leur position arrière maximale. Le clip arrière 2′ est disposé dans le support 1 ; ses tétons latéraux 31 sont positionnés dans les rainures correspondantes 35 et 36 et son appendice d'extrémité arrière est encastré d'une part, dans la rainure 37 et, d'autre part, entre les deux butées 38 du poussoir 5. Le clip avant 2″ est positionné dans le Vé porteur ; chacun de ses bras pinceurs 7 et 8 est encastré dans l'une des extensions latérales 3 et 4 et la mâchoire est dans une position d'ouverture maximum. Les tétons 31 du clip 2″ sont encastrés dans les rainures 35 et 36, au niveau du point de divergence maximal de ces dernières.

Après avoir repéré et saisi l'ovaire à traiter, l'opérateur positionne le manche 1 de telle sorte que les bras du premier clip 2″ enserrent le pédicule ovarien. L'opérateur peut alors, de sa main libre extérieure, actionner le poussoir 5 au moyen de la gâchette 6 après avoir forcé la lèvre de sécurité 46. Cette manoeuvre entraîne la progression du clip 2″ vers l'extrémité du support 1 et le rapprochement progressif des deux bras pinceurs au moyen des rainures 35 et 36 qui convergent et qui suivent les guides 40 d'extrémité. La fermeture "automatique" du clip peut, par cette opération, être réalisée soit sur les deux crans, soit simplement sur le premier. Lorsque la gâchette 6 arrive en bout de course, les lèvres 54 du poussoir passent les butées 55 et le premier clip 2″ se dégage du manche. L'opérateur positionne la gâchette 6 sur la deuxième butée 56 ; le deuxième clip 2′ est en position d'attente dans le Vé porteur, ses bras pinceurs sont ouverts et il peut être posé sur le second pédicule ovarien selon une opération similaire.

Si la fermeture automatique des clips est simplemnent réalisée au premier crantage, la pose peut être terminée à la main, suivant la taille de l'ovaire, pour provoquer l'ischémie de ses canaux d'irrigation.

Une ouverture hémisphérique 62 réalisée au niveau de l'une des extensions latérales 3, 4 peut être prévue pour permettre à l'opérateur de définir sensitivement la position du clip 2 à l'extrémité du manche 1.

Les clips 2′ et 2″ et leur support 1 sont réalisés en un matériau plastique aisément stérilisable. Le matériel sera de préférence mis à la disposition des utilisateurs dans un sachet stérile et sera de préférence à usage unique.

Les signes de référence insérés après les caractéristiques techniques mentionnées dans les revendications ont pour seul but de faciliter la compréhension de ces dernières.

## Revendications

1.- Matériel de stérilisation d'animal femelle par ischémie et dégénérescence du tissu ovarien, caractérisé en ce qu'il comprend :
   – des moyens pour provoquer l'ischémie du pédicule ovarien, en forme de clip (2),
   – un instrument (1) de pose dudit clip, muni, à l'une de ses extrémités, d'une tête opératoire destinée à être prise par une main de l'opérateur, in situ, et, à son autre extrémité, de moyens de manoeuvre (5, 6) à distance du clip (2), actionnés par l'autre main de l'opérateur, depuis l'extérieur.

2.- Matériel de stérilisation selon la revendication 1, caractérisé en ce qu'il comprend au moins un clip (2) constitué de deux bras pinceurs (7, 8) reliés entre eux, à une de leurs extrémités, par une articulation (9) et munis, à leur autre extrémité, de moyens de solidarisation (11, 12, 13, 14) destinés au maintien dudit clip en position fermée de serrage, lequel clip (2) est associé à un instrument de pose comprenant un support allongé (1) en forme de manche, lequel manche (1), d'une part, est muni à l'une de ses extrémités, de moyens de maintien du clip (2) en position ouverte, prêt à être positionné autour du pédicule ovarien et, d'autre part, présente une longueur L adaptée pour permettre à l'opérateur de réaliser de l'extérieur, la manoeuvre qui consiste à amener le clip (2) jusqu'au pédicule ovarien à traiter.

3.- Matériel de stérilisation selon l'une quelconque des revendications 1 ou 2, caractérisé en ce que la tête du manche définit un renflement d'extrémité en forme de Vé, lequel Vé est constitué par deux extensions latérales (3, 4) divergentes maintenant chacune un bras (7, 8) du clip (2).

4.- Matériel de stérilisation selon l'une quelconque des revendications 1 à 3, caractérisé en ce qu'il comporte un manche (1) creux dans lequel est guidé un poussoir (5) commandé de l'extérieur, lequel poussoir (5) est apte à au moins amorcer le positionnement du clip (2) sur l'organe à traiter.

5.- Matériel de stérilisation selon la revendication 4, caractérisé en ce que les extensions latérales (3, 4) qui définissent la tête du manche (1) comportent des paires de lèvres (34) qui prolongent la cavité interne dudit manche (1) et qui servent d'organes de guidage et d'encastrement des bras (7, 8) du clip (2), l'extrémité desdites extensions (3, 4) étant munie de guides (40) en forme de came servant à la commande de la fermeture des bras (3, 4) du clip (2) asservi au poussoir (5).

6.- Matériel de stérilisation selon l'une quelconque des revendications 4 ou 5, caractérisé en ce que le poussoir (5) est commandé de l'extérieur au moyen d'une gâchette (6) en saillie disposée au niveau de l'extrémité du manche (1) opposée à la tête support de clip (2), laquelle gâchette (6) coopère avec un système de prise, en forme de butées fixes (53) sur le manche (1), pour la manoeuvre d'une seule main par l'opérateur.

7.- Matériel de stérilisation selon l'une quelconque des revendications 1 à 6, caractérisé en ce qu'il comporte deux clips (2', 2") positionnés l'un derrière l'autre, asservis au poussoir (5), lequel poussoir comporte une gâchette (6) de manoeuvre à deux positions, associée à des moyens anti-retour (54, 55) du poussoir (5) par rapport au manche (1).

8.- Matériel de stérilisation selon l'une quelconque des revendications 1 à 7, caractérisé en ce que le manche (1) et le ou les clips (2', 2") associés, sont réalisés par moulage de matériau plastique biocompatible, du genre PVC, la longueur du manche (1) étant de l'ordre de 30 à 80 cm.

9.- Matériel de stérilisation selon l'une quelconque des revendications 1 à 8, caractérisé en ce que la tête du support (1) est associée à un perforateur (57) amovible, en forme de lame (58) montée sur une coiffe (59) qui s'emboîte sur l'extrémité dudit support.

10.- Matériel de stérilisation selon l'une quelconque des revendications 1 à 9, caractérisé en ce qu'il comporte une lèvre (46), moulée sur le support (1) et servant de cran de sécurité pour le mouvement du poussoir (5).

11.- Clip pour la stérilisation d'animal femelle par ischémie et dégénérescence du tissu ovarien, destiné à être adapté sur un instrument de pose selon l'une quelconque des revendications 1 à 10, comportant deux bras pinceurs (7, 8) reliés entre eux, à une de leurs extrémités, par une articulation (9) et munis, à leur autre extrémité, de moyens de solidarisation (11, 12, 13, 14) destinés au maintien dudit clip (2) en position fermée de serrage, lequel clip (2) comporte également, à chacune de ses extrémités, des moyens en forme de parois (10, 20) destinés à former avec les bras (7, 8) un espace clos dans lequel sont emprisonnés les tissus avant pincement.

12.- Clip de stérilisation selon la revendication 11, caractérisé en ce qu'il comporte une articulation flexible (9) en forme d'anneau, reliant l'une des extrémités des deux bras (7, 8), laquelle articulation (9) est associée à une languette (10) de blocage des tissus et éventuellement de rappel élastique pour forcer le maintien en position ouverte, l'autre extrémité de ces bras (7, 8) étant munie, pour l'un, d'appendices mâles (13), et pour l'autre, d'échancrures femelles (12) destinées à l'obtention d'une pluralité de crans de solidarisation, la zone médiane de ces moyens de solidarisation étant pourvue d'éléments mâles (15) et femelles (16) complémentaires, évitant le déport latéral des bras (7 et 8) l'un par rapport à l'autre.

**13.-** Clip de stérilisation selon la revendication 12, caractérisé en ce que les faces internes en vis à vis des bras (7, 8) sont munies de crans (17) transversaux,l'une desdites faces comportant une barrette (18) longitudinale faisant saillie, coopérant avec une échancrure (19) longitudinale prévue dans l'autre face pour former une chicane.

**14.-** Clip de stérilisation selon l'une quelconque des revendications 11 à 13, caractérisé en ce que la face interne de l'un des bras pinceurs (7) comporte des moyens d'emprisonnement et de blocage de la progression des tissus lors du pincement, en forme de butée (20) faisant saillie vers le bras (8) homologue, laquelle butée (20) est disposée légèrement en amont des moyens de solidarisation (11, 12, 13, 14) d'extrémité et coopère avec une réservation (23) de forme complémentaire, réalisée dans le bras homologue (8) pour permettre la fermeture.

**15.-** Clip de stérilisation selon la revendication 14, caractérisé en ce que la butée (20) présente une forme triangulaire, sa face arrière (21) étant destinée au blocage des tissus et sa face avant (22) comportant deux lèvres latérales (26) définissant un canal (27) dans lequel vient s'encastrer, lors du serrage des bras (7, 8) une lèvre médiane (28) prévue dans la réservation (23).

**16.-** Clip de stérilisation selon l'une quelconque des revendications 11 à 15, caractérisé en ce que les extrémités libres des bras (7, 8) comportent des tétons latéraux (31) de guidage destinés à coopérer avec des rainures (35, 36) prévues à l'intérieur du manche (1) et de ses extensions latérales (3, 4).

**17.-** Clip de stérilisation selon l'une quelconque des revendications 11 à 16, caractérisé en ce qu'il comporte un appendice arrière (32), au niveau de son articulation (9), lequel appendice est destiné d'une part au guidage du clip (2', 2") dans une rainure (37) prévue dans le manche, et, d'autre part, à son blocage entre deux butées (38) prévues à l'extrémité du poussoir (5).

_fig.1_

_fig.2_

_fig.3_

_fig.4_

_fig.6_     _fig.8_     _fig.7_

_fig.5_

_fig.10_

_fig.9_     _fig.11_

Office européen
des brevets

**RAPPORT DE RECHERCHE EUROPEENNE**

Numéro de la demande

EP    91 40 1053

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,D | EP-A-106 748 (US SURGICAL CORP.)<br>* page 6, ligne 7 - ligne 19 *<br>* page 13, ligne 12 - ligne 24 *<br>* revendication 1; figures 9-11 *<br>--- | 1-5,7,8 | A61D1/06<br>A61F6/20 |
| A | GB-A-2 054 384 (TREWAVIS)<br>* page 1, ligne 98 - ligne 100 *<br>--- | 6 | |
| X | GB-A-2 025 511 (HOLLISTER INC.)<br>* page 2, ligne 9 - ligne 85; figures 1,2 *<br>--- | 11-14 | |
| A | WO-A-8 001 752 (LEVEEN H. & LEVEEN E.)<br>* page 3, ligne 36 - page 4, ligne 5; figure 2 *<br>--- | 14,15 | |
| A | WO-A-8 602 541 (MATTSON)<br>* figure 22 *<br>--- | 16 | |
| A,D | AU-B-513 047 (BIGGERS)<br>* revendication 1 *<br>--- | 1 | |
| A | GB-A-1 020 821 (HUSTIN)<br>* page 3, ligne 43 - ligne 70 *<br>--- | 1 | DOMAINES TECHNIQUES RECHERCHES (Int. Cl.5) |
| A | EP-A-128 011 (ETHICON)<br>* page 5, ligne 31 - page 6, ligne 26 *<br>--- | 11 | A61D<br>E05B<br>A61F |
| A | US-A-4 800 879 (LERNER ET AL)<br>* colonne 4, ligne 6 - ligne 12; figure 2 *<br>--- | 11 | A61B |
| A | US-A-3 827 277 (WESTON)<br>* colonne 3, ligne 54 - colonne 4, ligne 16; figure 2 *<br>----- | 1 | |

Le présent rapport a été établi pour toutes les revendications

| Lieu de la recherche | Date d'achèvement de la recherche | Examinateur |
|---|---|---|
| LA HAYE | 01 AOUT 1991 | VLECK J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)